# FASCICULE DE BREVET EUROPEEN

(11) **EP 0 395 473 B1**
(45) Date de publication et mention de la délivrance du brevet: **28.12.1994**
(21) Numéro de dépôt: 90401052.7
(22) Date de dépôt: 18.04.1990
(51) Int. Cl.: A61B 5/08, A61B 7/04

(54) **Procédé et dispositif de diagnostic et rééducation orthophoniques**
Verfahren und Diagnosegerät orthophonischer Umschulung
Process and diagnostic device of orthophony reeducation

(30) Priorité: 26.04.1989 FR 8905545
(43) Date de publication de la demande: 31.10.1990
(73) Titulaire: S.A. SOREFAC, F-44200 Nantes (FR)
(72) Inventeur: Rineau, Georges, F-44200 Nantes (FR)
(74) Mandataire: Dawidowicz, Armand

(56) Documents cités:
- DE-A- 3 044 639
- DE-A- 3 044 639
- FR-A- 2 537 429
- FR-A- 2 575 917
- US-A- 3 752 929

## Description

L'invention concerne un procédé et un dispositif de diagnostic et de rééducation orthophoniques par contrôle visuel du souffle nasal et de l'émission des sons de la parole, du type comprenant un capteur du souffle nasal et un capteur buccal, ainsi que des moyens d'affichage des valeurs mesurées par lesdits capteurs.

On a proposé de nombreux procédés et dispositifs pour mesurer le souffle nasal, essentiellement dans le but de déterminer la présence d'insuffisances ou de fentes au niveau du palais ou de la cloison nasale.

On a ainsi proposé (voir introduction de FR-A-2.537.429) de détecter les vibrations de la cloison nasale et, simultanément, les ondes acoustiques émises par la bouche du patient, et de comparer les signaux ainsi émis. Ce procédé connu ne permet pas de mesurer de manière sûre le degré de nasalité dans tous les cas, en particulier pour le cas d'un palais légèrement fendu. En outre, la détection des vibrations de la cloison nasale est faussée par la distance entre le détecteur et la cloison, de sorte que le résultat est inexact.

FR-A-2.537.429, pour éliminer ces défauts, propose de mesurer les débits d'air exhalés respectivement par le nez et par la bouche. Pour ce faire, un masque combiné pour le nez et la bouche doit être appliqué sur le visage avec une séparation étanche entre les deux organes. On conçoit qu'un tel appareil, dont les résultats sont d'ailleurs contestables, ne puisse pas être utilisé pour un enfant en bas âge alors que c'est chez le bébé que la détection des insuffisances doit le plus souvent être détectée. En outre, un tel masque ne peut être utilisé pour la rééducation car il ne peut être supporté très longtemps par un patient.

Un masque semblable a été proposé dans US-A-3.752.929, l'analyse étant faite par deux microphones, un pour le nez et un pour la bouche. En plus des inconvénients précédents, la cloison séparant les deux microphones ne doit pas laisser passer les sons. Une telle condition est pratiquement impossible.

Dans US-A-3.906.936, on a proposé de mesurer le débit d'air nasal au moyen d'une thermistance montée dans un masque et plus ou moins refroidie par le flux d'air émis. Ce procédé de mesure connu présente les mêmes inconvénients que ceux indiqués précédemment.

La présente invention vise en conséquence à fournir un procédé et un dispositif du type décrit à l'introduction qui permettent une détection rapide et sûre du souffle nasal pendant la parole, aux moments précis des émissions vocales et en dehors des temps respiratoires, sans perturbation de l'observation du souffle nasal par le souffle respiratoire, et qui permettent une utilisation prolongée, éventuellement autonome, dans un but de rééducation.

A cet effet, le procédé selon l'invention est caractérisé par le fait qu'on place devant chaque narine un conduit muni d'une thermistance, on place devant la bouche un microphone, et on affiche séparément sous forme analogique les valeurs mesurées par les thermistances et par le microphone.

Du fait que l'analyse du souffle nasal est faite par un procédé différent de celui utilisé pour l'analyse du souffle buccal, il n'y a pas interférence entre les valeurs mesurées et affichées. L'affichage instantané sous forme analogique permet à l'opérateur d'effectuer un diagnostic immédiat par la prononciation de quelques sons de test et permet au patient de reconnaître ses propres défauts et de les corriger.

Le dispositif selon l'invention est caractérisé par le fait qu'il comprend un support ou boîtier muni de deux conduits dont la sortie débouche en regard de chacune des narines respectivement, et d'un microphone placé en regard de la bouche, chaque conduit étant muni d'une thermistance, le dispositif comprenant en outre des moyens de mesure et d'affichage du courant traversant les thermistances et des moyens de mesure et d'affichage de l'amplitude des sons reçus par le microphone.

Avantageusement, le dispositif comprend des moyens d'élimination sélective des valeurs lues par l'une ou l'autre des thermistances, afin de vérifier individuellement le souffle émis par chaque narine. Ces moyens d'élimination sélective peuvent comprendre un interrupteur de coupure de la thermistance.

Selon une forme de réalisation de l'invention, le dispositif comprend en outre des moyens d'enregistrement sonore stéréophonique et/ou graphique des valeurs mesurées.

Le dispositif de l'invention, qui est plus un dispositif de comparaison qu'un dispositif de mesure exacte, permet de traduire en mesures arbitraires sur un cadran ou vumètre la présence ou l'absence de souffle nasal pendant la parole qui est analysée simultanément pour en assurer le contrôle. Le dispositif permet d'effectuer ces mesures pour les deux narines simultanément ou pour chaque narine séparément. L'intensité de l'émission orale laryngée ou chuchotée étant reproduite simultanément sur un afficheur ou vumètre, le patient et/ou l'utilisateur peuvent établir une étude comparative entre le souffle nasal et l'émission orale.

L'utilisation d'un support commun pour les conduits nasaux et le microphone permet une utilisation prolongée pour la rééducation, ce support étant simplement placé devant le visage.

Le dispositif peut en outre comprendre un second support identique au précédent et utilisé par l'éducateur.

L'invention sera bien comprise à la lecture de la description suivante faite en se référant au dessin annexé dans lequel :

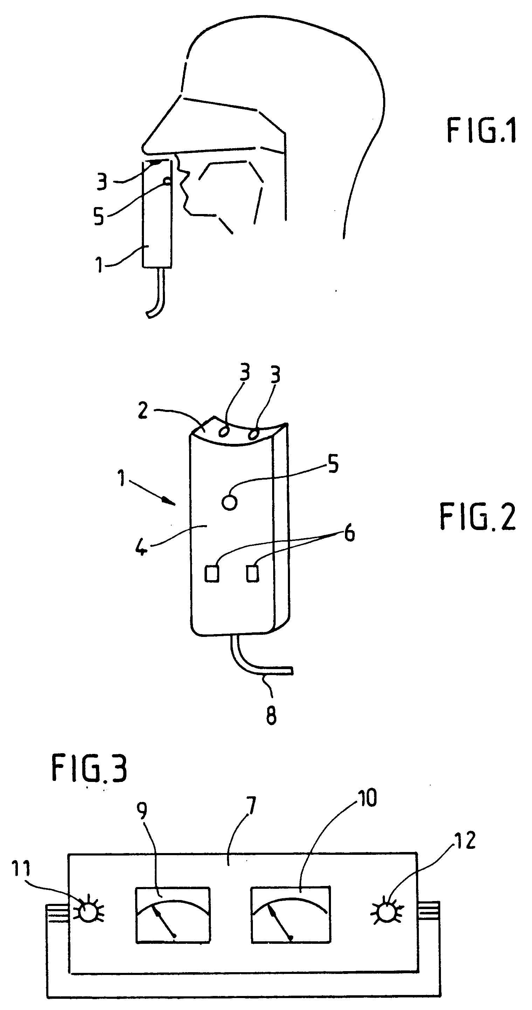

La figure 1 est un schéma d'un support d'un dispositif selon un exemple de réalisation de l'invention, en position d'utilisation ; la figure 2 est une vue schématique en perspective à plus grande échelle du support de la figure 1 et ; la figure 3 est une vue schématique en plan d'une console du dispositif.

Le dispositif comprend un support ou boîtier 1 comprenant sur une petite face 2, légèrement concave, les extrémités 3 de deux conduits. Sur une grande face 4, le boîtier 1 comporte un microphone piézo-électrique 5 et deux interrupteurs 6. La disposition est telle que, comme représenté à la figure 1, les extrémités 3 des conduits peuvent être placées respectivement sous chaque narine d'une personne, le microphone 5 se trouvant simultanément en face de sa bouche.

Les conduits se terminant aux extrémités 3 contiennent chacun un fil résistant ou thermistance alimenté en courant continu et un circuit de mesure de la tension aux bornes du fil. Cette tension varie avec la température du fil, laquelle est modifiée par le débit d'air sortant de la narine en regard. La tension mesurée est donc une fonction du souffle nasal.

Le boîtier 1 est relié à une console 7 de l'appareil (figure 3) par un cordon 8. Ce cordon 8 contient les fils d'alimentation des thermistances et du microphone 5, à partir d'une source électrique ou d'un transformateur ou redresseur contenu dans la console 7. Le circuit de mesure et le microphone 5 sont reliés à un dispositif électronique également contenu dans la console 7 et qui commande un vumètre 9 d'affichage analogique du souffle nasal mesuré par les thermistances et un vumètre 10 d'affichage analogique de l'amplitude de la parole, après redressement. Des boutons 11 et 12 permettent de régler respectivement la sensibilité des vumètres 9 et 10. Les interrupteurs 6 servent respectivement à mettre hors-circuit l'une ou autre des thermistances.

La console 7 peut comprendre une entrée pour un deuxième boîtier 1, une sortie d'enregistrement sonore stéréophonique, une sortie d'enregistrement graphique.

Le dispositif comporte, en outre un émetteur d'un signal sonore qui se déclenche en synchronisation précise avec le déplacement de l'aiguille du vumètre nasal pour signaler au sujet rééduqué la présence d'une fuite nasale pendant une lecture à haute voix.

Le dispositif qui vient d'être décrit permet de détecter certaines anomalies telles que rhinolalies ouvertes et fermées, insuffisances vélaires, nasonnement, obstructions nasales, émissions incontrôlées dans la surdité. Il permet également d'éduquer ou de rééduquer ces mêmes troubles phonétiques.

On peut prévoir dans le cadre de la rééducation, de restituer dans un casque ou similaire l'émission parlée du sujet testé et captée par le microphone, lorsque cette émission est dénuée de toute nasalité.

Il est également possible d'adjoindre un enregistrement des vibrations laryngées au moyen d'un capteur disposé sur le cartilage thyroïde et de l'afficher sur une échelle d'intensité étalonnée, afin d'affiner la détection des malformations.

## Revendications

1. Procédé de détection de malformations et de rééducation orthophoniques par contrôle visuel du souffle nasal et des sons de la parole,
caractérisé par le fait qu'on place devant chaque narine un conduit (3) muni d'une thermistance, on place devant la bouche un microphone (5), et on affiche séparément sous forme analogique les valeurs mesurées par les thermistances et par le microphone (3).

2. Dispositif du type comprenant un capteur du souffle nasal et un capteur buccal, ainsi que des moyens d'affichage des valeurs mesurées par lesdits capteurs pour la mise en oeuvre du procédé selon la revendication 1,
caractérisé par le fait qu'il comprend un support ou boîtier (1) muni de deux conduits dont la sortie (3) débouche en regard de chacune des narines respectivement, et d'un microphone (5) placé en regard de la bouche, chaque conduit étant muni d'une thermistance, le dispositif comprenant en outre des moyens de mesure et d'affichage (7, 9) du courant traversant les thermistances et des moyens de mesure et d'affichage (7, 10) de l'amplitude des sons reçus par le microphone (5).

3. Dispositif selon la revendication 2,
caractérisé par le fait qu'il comprend des moyens d'élimination sélective (6) des valeurs mesurées par l'une ou l'autre des thermistances.

4. Dispositif selon l'une des revendications 2 et 3,
caractérisé par le fait qu'il comprend en outre des moyens d'enregistrement sonore stéréophonique et/ou graphique des valeurs mesurées.

5. Dispositif selon l'une des revendications 2 à 4,
caractérisé par le fait qu'il comprend un second support ou boîtier identique au support ou boîtier (1).

6. Dispositif selon l'une des revendications 2 à 5,
caractérisé par le fait qu'il comprend des moyens (11, 12) de réglage de la sensibilité de mesure.

7. Dispositif selon l'une des revendications 1 à 6,
caractérisé par le fait qu'il comporte un émetteur de signal sonore pour avertir de ses déficiences phonétiques le lecteur pendant la lecture à haute voix.

8. Dispositif selon l'une des revendications 1 à 7,
caractérisé par le fait qu'il comprend des moyens d'audition, tels qu'un casque, des bruits captés par ledit microphone.

9. Dispositif selon l'une des revendications 1 à 8,
caractérisé par le fait qu'il comprend un capteur des vibrations laryngées relié à un affichage sur une échelle d'intensité étalonnée.

## Claims

1. A method of detecting erroneous formations and for re-education in speech therapy, by checking visually the nasal breath and sounds of the spoken word, characterized in that a duct (3) fitted with a temperature-sensitive resistor is placed in front of each nostril, a microphone (5) is placed in front of the mouth and the values measured by the temperature-sensitive resistors and the microphone (3) are displayed separately in analog form.

2. A device of the type comprising a sensor for nasal breath and an oral sensor, as well as means for displaying the values measured by the said sensors, for implementing the method according to claim 1, characterized in that it comprises a support or box (1) provided with two ducts, whose outlets (3) open opposite the nostrils respectively, and a microphone (5) located opposite the mouth, each duct being fitted with a temperature-sensitive resistor, the device further comprising means (7, 9) for measuring and displaying the current passing through the temperature-sensitive resistors and means (7, 10) for measuring and displaying the amplitude of sounds received by the microphone (5).

3. A device according to claim 2, characterized in that it comprises means (6) for selective elimination of the values measured by one or the other of the temperature-sensitive resistors.

4. A device according to claim 2 or 3, characterized in that it further comprises means for stereophonic audio recording and/or graphical recording of measured values.

5. A device according to any of claims 2 to 4, characterized in that it comprises a second support or box identical to the support or box (1).

6. A device according to any of claims 2 to 5, characterized in that it comprises means (11, 12) for adjusting the measuring sensitivity.

7. A device according to any of claims 1 to 6, characterized in that it comprises a sound signal emitter for informing the reader of his phonetic ertors during reading out loud.

8. A device according to any of claims 1 to 7, characterized in that it comprises listening means, such as a helmet, for the noises detected by the said microphone.

9. A device according to any of claims 1 to 8, characterized in that it comprises a sensor for vibrations of the larynx connected to a display on a standardised intensity scale.

## Patentansprüche

1. Verfahren zur Entdeckung von Fehlbildungen und zur orthophonischen Umschulung durch visuelle Kontrolle der Luftströmung durch die Nase und der Klänge der Sprache, **dadurch gekennzeichnet**, daß man vor jedem Nasenloch einen Leiter (3), der mit einem Thermistor versehen ist, anordnet, daß man vor dem Mund ein Mikrophon (5) anordnet und daß man getrennt voneinander in analoger Form die durch die Thermistoren und durch das Mikrophon (3) gemessenen Werte anzeigt.

2. Vorrichtung zur Durchführung des Verfahrens nach Anspruch 1, mit einem Aufnehmer für die Luftströmung durch die Nase und einen Aufnehmer für den Mund, sowie mit Mitteln zum Anzeigen der durch genannte Aufnehmer gemessenen Werte,
**dadurch gekennzeichnet**, daß sie eine Halterung oder ein Gehäuse (1) aufweist, das mit zwei Leitern versehen ist, deren Austritt (3) gegenüber jeweils einem der Nasenlöcher mündet, und das mit einem Mikrophon (5), das gegenüber dem Mund angeordnet ist, versehen ist, wobei jeder der Leiter mit einem Thermistor versehen ist und wobei die Vorrichtung ferner Mittel zur Messung und Anzeige (7,9) des Stromes, der die Thermistoren durchfließt, und Mittel zur Messung und Anzeige (7,10) der Amplitude der Klänge, die durch das Mikrophon (5) empfangen werden, aufweist.

3. Vorrichtung nach Anspruch 2, **dadurch gekennzeichnet**, daß sie Mittel (6) zur wahlweisen Ausschaltung der durch den einen oder den anderen der Thermistoren gemessenen Werte aufweist.

4. Vorrichtung nach einem der Ansprüche 2 und 3, **dadurch gekennzeichnet**, daß sie ferner Mittel zur stereophonen Ton- und/oder graphischen Aufzeichnung der gemessenen Werte aufweist.

5. Vorrichtung nach einem der Ansprüche 2 bis 4, **dadurch gekennzeichnet**, daß sie eine zweite Halterung oder ein zweites Gehäuse aufweist, das der Halterung oder dem Gehäuse (1) gleicht.

6. Vorrichtung nach einem der Ansprüche 2 bis 5, **dadurch gekennzeichnet**, daß sie Mittel (11, 12) zur Einstellung der Empfindlichkeit der Messung aufweist.

7. Vorrichtung nach einem der Ansprüche 1 bis 6, **dadurch gekennzeichnet**, daß sie einen Sender für Tonsignale aufweist, um den Leser während des Lesens mit lauter Stimme über seine phonetischen Unzulänglichkeiten zu unterrichten.

8. Vorrichtung nach einem der Ansprüche 1 bis 7, **dadurch gekennzeichnet**, daß sie Mittel zum Hören der durch genanntes Mikrophon aufgenommenen Geräusche, beispielsweise einen Kopfhörer, aufweist.

9. Vorrichtung nach einem der Ansprüche 1 bis 8, **dadurch gekennzeichnet**, daß er einen Aufnehmer für Kehlkopfschwingungen aufweist, der an einen Anzeiger mit einer geeichten Skala angeschlossen ist.
